# EUROPÄISCHE PATENTSCHRIFT

(11) **EP 0 410 199 B1**
(45) Veröffentlichungstag und Bekanntmachung des Hinweises auf die Patenterteilung: **10.05.1995**
(21) Anmeldenummer: 90113120.1
(22) Anmeldetag: 10.07.1990
(51) Int. Cl.: C08F 283/00, A61K 6/08

(54) **Mehrstufig härtende Kunststoffe**
Plastic material curable in multiple stages
Matériau en plastique durcissable en plusieurs étapes

(30) Priorität: 22.07.1989 DE 3924308
(43) Veröffentlichungstag der Anmeldung: 30.01.1991
(73) Patentinhaber: BAYER AG, 51368 Leverkusen (DE)
(72) Erfinder: Winkel, Jens, Dr., D-5000 Köln 71 (DE); Schwabe, Peter, Dr., D-5090 Leverkusen (DE); Müller, Hanns-Peter, Dr., D-5060 Bergisch Gladbach (DE)

(56) Entgegenhaltungen:
- EP-A- 0 127 758
- EP-A- 0 347 711
- DE-A- 3 636 974

## Beschreibung

Die Erfindung betrifft neuartige, in zwei Stufen aushärtende Kunststoffe; das sind Materialien, die - ausgehend von einem flüssigen Zustand - kontrolliert in einen elastischen Körper überführt werden können, der anschließend zu einem harten, widerstandfähigen Material ausgehärtet werden kann. Solche Materialien sind u.a. im Dentalbereich, z.B. zur Herstellung künstlicher Zähne oder Zahnteile, von Interesse.

Mehrstufig aushärtende Systeme sind im Prinzip bekannt. So beschreibt die DE-PS 3 506 020 ein Verfahren zur Herstellung künstlicher Zähne oder Zahnteile, bei dem Diisocyanate mit Polyolen in Anwesenheit von Methacrylatmonomeren zu Polyurethangelen umgesetzt werden, die anschließend durch eine radikalische Polymerisation ausgehärtet werden können. Derartige Systeme haben den Nachteil, daß die Bildung des Polyurethangels unter Ausschluß von Feuchtigkeit erfolgen muß, um unerwünschte Nebenreaktionen zu vermeiden und sind daher für eine Anwendung in feuchter Umgebung, wie sie z.B. bei Anwendung im Mund gegeben ist, ungeeignet.

Weiterhin bekannt sind Kunststoffe zur Herstellung für provisorischen Zahnersatz, wie sie z.B. in der Dissertation von H. Frey beschrieben sind (H. Frey: Physikalische Eigenschaften verschiedener Kunststoffe für provisorischen Zahnersatz in der Kronen- und Brückenprothetik, Zürich 1980, G. Bohi, Buch- und Offsetdruck, Zürich) und werden in der Zahlheilkunde vielfach verwendet. Die Systeme bestehen aus einem Polymethacrylat Pulver, das in niedermolekularen Methacrylsäure-Estern angequollen wird. Dieses Gel kann anschließend radikalisch ausgehärtet werden. Um eine ausreichende Quellungsgeschwindigkeit zu erreichen, müssen niedermolekulare Methacrylsäureester verwendet werden, die sehr stark riechen und häufig zu allergischen Reaktionen führen.

Es wurde nun gefunden, daß Mischungen aus Silikopolyethern, wie sie in der DE-PS 3 636 924 beschrieben werden, und radikalisch härtenden Monomeren unter Verwendung mindestens zweier geeigneter Katalysatoren in zwei Stufen ausgehärtet werden können. In der ersten Stufe reagiert dabei der Silikopolyether in Anwesenheit von Säuren zu einem Gel, das in einem zweiten Schritt durch Radikalinitiatoren zu einem harten, widerstandsfähigen Material polymerisiert werden kann. Die erfindungsgemäßen Materialien sind allgemein dadurch gekennzeichnet, daß sie
a) mindestens einen polyfunktionellen Silikopolyether,
b) mindestens ein radikalisch härtendes Monomer,
c) mindestens einen Katalysator für die Heiß-, Kalt- oder Lichtpolymerisation der radikalischen Monomeren und
d) mindestens einen Katalysator zur Kondensation des Silikopolyethers
enthalten.

Bevorzugt betrifft die Erfindung mehrstufig aushärtbare Kunststoffe, bestehend aus Ether-, Urethan- und Harnstoffgruppen enthaltenden Polyadditionsprodukten mit Alkoxysilylendgruppen einer überwiegend linearen Molekülstruktur mit ausschließlich aliphatisch oder cycloaliphatisch gebundenen Ether-, Urethan- und Harnstoffsegmenten und einem mittleren Molekulargewicht Mn von 800 - 20 000,
gekennzeichnet durch
a) einen Gehalt an Polyethergruppen von 25 bis 90 Gew.-Teilen, vorzugsweise von 50 bis 80 Gew.-Teilen auf 100 Gew.-Teile Polyadditionsprodukt,
b) einen Gehalt an Urethangruppen 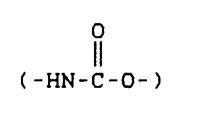 von 0,5 bis 10 Gew.-Teilen, vorzugsweise von 1 bis 8 Gew.-Teilen auf 100 Gew.-Teile Polyadditionsprodukt,
c) einen Gehalt an Harnstoffgruppen 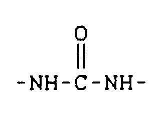 von 0,5 bis 10 Gew.-Teilen, vorzugsweise 1 - 8 Gew.-Teilen auf 100 Gew.-Teile Polyadditionsprodukt und
d) einen Gehalt an endständigen Alkoxysilylgruppen 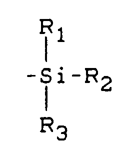 von 1 bis 25 Gew.-Teilen, vorzugsweise 2 bis 10 Gew.-Teilen auf 100 Gew.-Teile Polyadditionsprodukt, wobei die Alkoxysilylgruppen im Polyaddukt über folgende Verbindungen eingebracht werden und folgende Formel aufweisen: 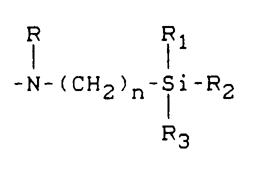 worin
   - n: die Zahlen 1 bis 6, bevorzugt die Zahl 3 darstellt,
   - R: Wasserstoff oder -(CH₂)ₙ-SiR₁R₂R₃ bedeutet,
   - R₁: C₁-C₄-Alkoxy, bevorzugt Methoxy oder Ethoxy bedeutet,
   - R₂ und R₃: die gleiche Bedeutung wie R₁ aufweisen und zusätzlich die Methyl- oder Ethylgruppe darstellen.

Bevorzugt betrifft die Erfindung mehrstufig aushärtbare Kunststoffe, bestehend aus mindestens einem Silikopolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers.

Die erfindungsgemäß verwendbaren Silikopolyether können hergestellt werden, indem man aliphatische und/ oder cycloaliphatische Diisocyanate mit Dihydroxypolyethern des mittleren Molgewichtsbereiches Mn von 300 bis 6000 umsetzt, wobei man gegebenenfalls zusätzlich noch aliphatische und/oder cycloaliphatische zweiwertige Alkohole eines mittleren Molgewichtes Mn von 62 bis < 300 zusetzt, und die erhaltenen Prepolymeren mit Alkoxysilylmonoaminen umsetzt, wobei man gegebenenfalls noch aliphatisch und/oder cycloaliphatische Diamine mit primären Aminogruppen mit einem Molekulargewicht Mn von 60 bis 300 mitverwenden kann, indem man
a) Alkoxysilylmonoamine der Formel

   HRN-(CH₂)ₙ-SiR₁R₂R₃

   mit den oben näher bezeichneten Bedeutungen zusetzt, wobei man ferner
b) pro 1 Gew.-Tl. Dihydroxypolyether
   - 0,05 bis 1,5, bevorzugt 0,1 bis 0,5 Gew.-Tl. des Diisocyanates,
   - 0 bis 0,6, bevorzugt 0 bis 0,2 Gew.-Tl. des zweiwertigen Alkoholes,
   - 0,02 bis 0,40, bevorzugt 0,05 bis 0,2 Gew.-Tl. des Alkoxysilylmonoamins und
   - 0 bis 0,6, bevorzugt 0 bis 0,2 Gew.-Tl. des Diamins,
einsetzt.

Die Umsetzung der Komponenten erfolgt bei Temperaturen von 20 bis 150° C, bevorzugt von 60 bis 120° C.

Die gegebenenfalls eingesetzten Diamine dienen zur Einstellung des jeweils gewünschten Molekulargewichtes.

Geeignete Diisocyanate sind insbesondere solche mit aliphatisch und/oder cycloaliphatisch gebundenen Isocyanatgruppen der Formel Q(NCO)₂, in welcher Q für einen aliphatischen Kohlenwasserstoffrest mit 2 bis 12 Kohlenstoffatomen oder einen cycloaliphatischen bzw. gemischt aliphatisch-cycloaliphatischen Kohlenwasserstoffrest mit 4 bis 15 Kohlenstoffatomen steht.

Beispiele derartiger Diisocyanate sind Ethylendiisocyanat, Tetramethylendiisocyanat, Hexamethylendiisocyanat, Dodecamethylendiisocyanat, Cyclobutan-1,3-diisocyanat, Cyclohexan-1,3- und 1,4-diisocyanat oder 1-Isocyanato-3,3,5-trimethyl-5-isocyanatomethyl-cyclohexan bzw. beliebige Gemische derartiger Diisocyanate. Vorzugsweise werden beim erfindungsgemäßen Verfahren cycloaliphatische bzw. gemischt aliphatisch-cycloaliphatische Diisocyanate eingesetzt. Besonders bevorzugt ist 1-Isocyanato-3,3,5-trimethyl-5-isocyanato-methyl-cyclohexan (Isophorondiisocyanat).

Geeignete Dihydroxypolyether sind ebenfalls solche der an sich bekannten Art und werden z.B. durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von BF₃, oder durch Anlagerung dieser Epoxide, gegebenenfalls im Gemisch oder nacheinander, an Startkomponenten mit reaktionsfähigen Wasserstoffatomen wie Alkohole oder Amine, z.B. Wasser, Ethylenglykol, Propylenglykol-(1,3) oder -(1,2), 4,4′-Dihydroxydiphenylpropan, Anilin, hergestellt. Vielfach sind solche Polyether bevorzugt, die überwiegend (bis zu 90 Gew.-%, bezogen auf alle vorhandenen OH-Gruppen im Polyether) primäre OH-Gruppen aufweisen.

Geeignete Diamine sind vorzugsweise primäre Aminogruppen aufweisende aliphatische, cycloaliphatische oder gemischt aliphatisch-cycloaliphatische Diamine des Molekulargewichtsbereichs 60 bis 300. Beispiele sind Ethylendiamin, Tetramethylendiamin, Hexamethylendiamin, 4,4′-Diamino-dicyclohexylmethan, 1,4-Diaminocyclohexan, 4,4′-Diamino-3,3′-dimethyl-dicyclohexylmethan oder 1-Amino-3,3,5-trimethyl-5-aminomethyl-cyclohexan (Isophorondiamin). Ganz besonders bevorzugt werden 4,4′-Diaminodicyclohexylmethan oder das zuletzt genannte Isophorondiamin eingesetzt.

Als zweiwertige Alkohole kommen z.B. Ethylenglykol, Propylenglykol-(1,2) und -(1,3), Butylenglykol-(1,4) und -(2,3), Hexandiol-(1,6), Octandiol-(1,8), Neopentylglykol, Cyclohexandimethanol, 1,4-Bis-hydroxymethyl-cyclohexan, 2-Methyl-1,3-propandiol, 3-Methylpentandiol-(1,5), ferner Diethylenglykol, Triethylenglykol, Tetraethylenglykol, Polyethylenglykole, Dipropylenglykol, Polypropylenglykole, Dibutylenglykole und Polybutylenglykole in Frage.

Geeignete Monoamine sind ebenfalls bekannt, hierbei handelt es sich vorzugsweise um die technisch gut zugänglichen γ-Aminopropyl-tri-C₁-C₄-alkoxysilane bzw. um Bis-(3-C₁-C₄-alkoxysilylpropyl)-amine, ganz besonders bevorzugt ist γ-Aminopropyl-triethoxysilan.

Erfindungsgemäß geeignet sind auch Abmischungen des geschilderten Polyadditionsproduktes mit einem Vernetzungsmittel, wobei als Vernetzungsmittel bevorzugt Tetrakieselsäureester, insbesondere Tetraethoxysilan, und Polyalkoxypolysiloxane verwendet werden.

Bezogen auf 1 Gewichtsteil des Polyadditionsproduktes können 0,01 bis 5, bevorzugt 0,1 bis 1 Gewichtsteile des Vernetzungsmittels eingesetzt werden.

Die Herstellung dieser Mischung erfolgt in einer dem Fachmann bekannten Weise, beispielsweise durch Mischen der Komponenten bei Raumtemperatur gegebenenfalls bei mäßig erhöhter Temperatur bis 60° C.

Als Comonomere eignen sich prinzipiell alle radikalisch härtbaren Monomere.

Insbesondere eignen sich hierfür die an sich bekannten Methacrylate in monofunktioneller oder polyfunktioneller Form, die allein oder in Mischungen eingesetzt werden können. Beispiele sind Methylmethacrylat, Isobutylmethacrylat, Cyclohexylmethacrylat, Ethylenglycoldimethacrylat, Diethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, aber auch Bis-GMA sowie die Reaktionsprodukte aus Isocyanaten, insbesondere Di- und/oder Triisocyanaten und OH-gruppenhaltigen Methacrylaten. Beispiele für letztere sind die Umsetzungsprodukte von 1 Mol Hexamethylendiisocyanat mit 2 Mol 2-Hydroxyethylmethacrylat, von 1 Mol Tris (6-isocyanatohexyl)isocyanurat mit 3 Mol Hydroxyethylmethacrylat, und von 1 Mol Trimethylhexamethylendiisocyanat mit 2 Mol Hydroxyethylmethacrylat. Der Anteil dieser Verbindungen in der Mischung mit dem/den Silikopolyether(n) bewegt sich zwischen 10 und 90 Gewichtsprozent, bevorzugt zwischen 50 und 80 Gewichtsprozent.

Die Verbindungen können mit an sich bekannten Katalysatoren ausgehärtet werden. So für die Heißpolymerisation mit Peroxiden wie Dibenzoylperoxid, Dilaurylperoxid, tert.-Butylperoktoat oder tert.-Butylperbenzoat, aber auch mit α,α-Azo-bis-(isobutyroethylester)-Benzpinakol und 2,2′-Dimethylbenzpinakol.

Es können aber auch Katalysatoren eingesetzt werden, die durch Strahlung aktivierbar sind. Die Verwendung von einem Photosensibilisator zusammen mit einem Reduktionsmittel wird bevorzugt. Beispiele für Photosensibilisatoren sind α-Diketone wie 9,10-Phenanthrenchinon, Diacetyl, Furil, Anisil, 4,4′-Dichlorbenzil und 4,4′-Dialkoxybenzil. Campherchinon wird bevorzugt verwendet. Beispiele für Reduktionsmittel sind Amine wie Cyanoethylmethylanilin, Dimethylaminoethylmethacrylat, n-Butylamin, Triethylamin, Triethanolamin, N,N-Dimethylanilin oder N-Methyldiphenylamin.

Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt.

Die peroxid- bzw. aminhaltigen Monomermischungen werden bis zur Anwendung getrennt gelagert.

Die erwähnten Katalysatoren werden in Mengen von 0,01 bis 10 Gew.-%, bezogen auf das polymerisierbare Material, verwendet, insbesondere in Mengen von 0,1 bis 5 Gew.-%.

Der elastische Zwischenzustand entsteht durch Polykondensation von Ether-, Urethan- oder Harnstoffgruppen enthaltenden Polyadditionsprodukten mit Alkoxysilylendgruppen, wie sie in der DE-PS 3 636 974 beschrieben sind. Die Silikopolyether können durch anorganische und/oder organische Säuren ausgehärtet werden Geeignet sind z.B. saure Ionentauscher, Phosphorsäure, Dibutylphosphorsäure, verdünnte Schwefelsäure, Weinsäure, Zitronensäure, Adipinsäure, Salzsäure, Sulfonsäuren usw.. Bevorzugt ist die Salzsäure.

Die Materialien können durch unterschiedliche Säuregehalte auf die gewünschte Aushärtungszeit eingestellt werden.

Je nach Verwendungszweck des Werkstoffes können die Materialien auch anorganische oder organische Füllstoffe enthalten. Geeignete anorganische Füllstoffe sind z.B.:

Bergkristall, Kristoballit, Quarzglas, hochdisperse Kieselsäure, Aluminiumoxid und Glaskeramiken, beispielsweise Lanthan und Zirkon enthaltende Glaskeramiken (DE-PS 2 347 591). Die anorganischen Füllstoffe werden zur Verbesserung des Verbunds zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297-308 (1983)). Bevorzugt wird 3-Methacryloyloxypropyl-trimethoxysilan eingesetzt. Es kann auch vorteilhaft sein, mehrere Füllstoffe nebeneinander einzusetzen, die einen voneinander verschiedenen Teilchendurchmesser und/oder einen unterschiedlichen Silangehalt besitzen.

Der Füllstoffanteil in der Mischung beträgt im allgemeinen 5 bis 80 Gew.-%, vorzugsweise 40 bis 70 Gew.-%. Dem Werkstoff können auch organische Polymere oder Copolymere zugesetzt werden. Weiterhin können auch die üblichen Hilfsstoffe wie Stabilisatoren, Lichtschutzmittel und Farbstoffe enthalten sein. Beispielhaft sei die folgende Zusammensetzung der Komponente A genannt.
100 Gewichtsteile der Komponente A enthalten:
a) 15 - 50 Gewichtsteile Silikopolyether
b) 40 - 70 Gewichtsteile polymerisierbare Vinylverbindungen
c) 0 - 70 Gewichtsteile Füllstoffe
d) 0,1 - 5 Gewichtsteile Initiator für die radikalische Polymerisation
e) 0 - 5 Gewichtsteile Hilfsstoffe
Beispielhaft sei die folgende Zusammensetzung der Komponente B genannt.
100 Gewichtsteile Komponente B enthalten:
f) 5 - 30 Gewichtsteile einer organischen oder anorganischen Säure
g) 0 - 10 Gewichtsteile Wasser
h) 0 - 70 Gewichtsteile Vinylverbindungen
i) 0 - 70 Gewichtsteile Füllstoff
Die folgenden Beispiele dienen der weiteren Erläuterung der Erfindung.

### Beispiel 1

800 g (0,4 Mol OH) eines linearen Polyethers des MG 4000 (hergestellt durch Polyaddition von 87 Gew.-Teilen Propylenoxid auf Propylenglykol und anschließender Polyaddition von 13 Gew.-Teilen Ethylenoxid) werden 30 Minuten bei 120° C im Wasserstrahlvakuum entwässert. Danach werden dem Ansatz 88,8 g (0,8 Mol NCO) Isophorondiisocyanat (im folgenden IPDI genannt) zugefügt und das Ganze 4 Stunden bei 120°C bis 140°C unter Stickstoff gerührt. Anschließend wird die NCO-Zahl des Prepolymeren bestimmt.
NCO gef.: 1,70 %
NCO ber.: 1,89 %
Zu dem Prepolymeren werden dann 88,4 g (0,4 Mol) 3-Aminopropyltriethoxysilan unter Rühren und Stickstoff bei 30° C innerhalb von 30 Minuten zugetropft. Dabei erwärmt sich die Mischung auf 50° C. Man läßt anschließend bei 60° C 30 Minuten nachreagieren. Im resultierenden Polyurethan-Polyharnstoff läßt sich IR-spektroskopisch kein NCO mehr nachweisen. Man erhält auf diese Weise nach Abkühlen ein fast farbloses, klares, viskoses Produkt mit einem Gehalt an -HN-CO-NH- von 1,237 Gew.-% und einem Gehalt an endständigen Alkoxysilylgruppen von 6,22 Gew.-%.

### Beispiel 2

1000 g (0,5 Mol OH) eines linearen Polyethers des MG 4000 (hergestellt durch Polyaddition von 70 Gew.-Teilen Propylenoxid auf Propylenglykol und anschließender Polyaddition von 30 Gew.-Teilen Ethylenoxid) werden wie in Beispiel 1 entwässert. Bei 40° C werden dem Ansatz 111 g (1 Mol NCO) IPDI in einem Guß zugegeben und anschließend 1 Tropfen Zinkoktoat. Unter Rühren und Stickstoff wird der Ansatz auf 110° C geheizt und 40 Minuten bei dieser Temperatur belassen. Anschließend wird die NCO-Zahl des Prepolymeren bestimmt.
NCO gef.: 1,69 %
NCO ber.: 1,89 %
Nach Abkühlen auf 60° C tropft man unter Rühren und Stickstoff 110,5 g (0,5 Mol) 3-Aminopropyl-triethoxysilan innerhalb von 10 Minuten ein. Dabei erwärmt sich der Ansatz auf 75° C. Man läßt den Ansatz ohne weitere Heizung 30 Minuten nachreagieren. Im resultierenden Polyurethan-Polyharnstoff läßt sich IR-spektroskopisch kein freies NCO mehr nachweisen. Der Polyurethan-polyharnstoff ist farblos, klar und gut gießfähig mit einem Gehalt an -HN-CO-NH- von 2,37 Gew.-% und einem Gehalt an endständigen Alkoxysilylgruppen von 6,22 Gew.-%.

### Beispiel 3

1000 g (0,5 Mol OH) eines linearen Polyethers des MG 2000 (hergestellt durch Polyaddition von gleichen Gew.-Teilen Propylenoxid und Ethylenoxid auf Propylenglykol) werden wie in Beispiel 1 entwässert. Bei 80° C werden dem Ansatz 166,5 g (1,5 Mol NCO) IPDI in einem Guß zugegeben und anschließend 1 Tropfen Zinkoktoat. Unter Stickstoff wird der Ansatz bei 120° C 4 Stunden gerührt. Anschließend wird die NCO-Zahl des Prepolymeren bestimmt.
NCO gef.: 1,79 %
NCO ber.: 1,80 %
Nach Abkühlen auf 60° C tropft man unter Rühren und Stickstoff 110,5 g (0,5 Mol) 3-Aminopropyl-triethoxysilan zu. Danach ist der Ansatz NCO-frei. Der Polyurethan-Polyharnstoff ist eine farblose, klare und hochviskose Flüssigkeit mit einem Gehalt an -HN-CO-NH-von 2,27 Gew.-% und einem Gehalt an endständigen Alkoxysilylgruppen von 5,95 Gew.-%.

### Beispiel 4

Man verfährt wie in Beispiel 3 beschrieben, nur mit dem Unterschied, daß dem resultierenden Polyurethan-polyharnstoff nach der Herstellung 639 g Tetraethoxysilan zugegeben werden. Die homogene Mischung ist klar, farblos und besitzt eine Viskosität 25° C von 2016 mPas.

Die gebrauchsfertige Mischung enthält 1,51 Gew.-% -HN-CO-NH- und 3,96 Gew.-% endständig gebundene Alkoxysilylgruppen und zusätzlich 50 Gew.-% Tetraethoxysilan.

### Beispiel 5

### a) Komponente A

In einer Mischung aus 40 Gewichtsteilen Silikopolyether (Beispiel 1) und 60 Gewichtsteilen Triethylenglycoldimethacrylat werden 0,2 Gewichtsteile Campherchinon, 0,1 Gewichtsteile 3,5-di-tert.-Butyl-4-hydroxitoluol und 0,5 Gewichtsteile 4-N,N-Dimethylaminobenzolsulfonsäure-diallylamid gelöst.

### b) Komponente B

4,1 Gewichtsteile 2-Hydroxyethylmethacrylat werden mit 3,5 Gewichtsteilen normaler Salzsäure gemischt.

Mischt man 10 Gewichtsteile der Komponente A mit 1 Gewichtsteil der Komponente B, so härtet die Mischung in 5 Minuten zu einem elastischen Körper aus.

Bestrahlt man diesen Körper mit einer handelsüblichen Dentallampe (z.B. LCU, Fa. Bayer) oder in einem handelsüblichen Lichtofen (z.B. Dentacolor XS, Fa. Kulzer), so härtet der Körper zu einem starren Material aus.

### Beispiel 6

### Basispaste

41,7 Gewichtsteile einer Lösung, bestehend aus 20 Gewichtsteilen Silikopolyether (aus Beispiel 2), 56 Gewichtsteilen 2,2-Bis-4-(3-methacryloxy-2-hydroxypropyl)phenyl-propan, 23,2 Gewichtsteilen Triethylenglycoldimethacrylat, 0,2 Gewichtsteilen Campherchinon, 0,1 Gewichtsteilen 3,5-di-tert.-Butyl-4-hydroxitoluol und 0,5 Gewichtsteilen 4-N,N-Dimethylaminobenzolsulfonsäurediallylamid werden mit 58,3 Gewichtsteilen eines nach Standardmethoden oberflächenbehandelten Glases (z.B. Glas GM 27884 k/6, Fa. Schott, mit einem C-Wert von 2,1 %) zu einer Paste verarbeitet.

Vermischt man 10 Teile dieser Basispaste mit 1 Gewichtsteil der Komponente B aus Beispiel 5, so härtet die Mischung in 10 Minuten zu einem elastischen Körper aus.

Bestrahlt man diesen Körper 60 sec. in einem handelsüblichen Lichtofen (z.B. Dentacolor XS, Fa. Kulzer), so erhält man einen Festkörper mit einer Biegefestigkeit von 62 N/mm², einem Biegemodul von 2400 N/mm² und einer Wasseraufnahme von 3,6 mg/cm².

### Beispiel 7

41,7 Gewichtsteile einer Lösung, bestehend aus 20 Gewichtsteilen Silikopolyether (aus Beispiel 2), 55 Gewichtsteilen 2,2-Bis-[4-(3-methacryloxy-2-hydroxypropyl)-phenyl]-propan, 22,9 Gewichtsteilen Benzoylperoxid und 0,1 Gewichtsteilen 3,5-di-tert.-Butyl-4-hydroxitoluol werden mit 58,3 Gewichtsteilen eines nach Standardmethoden oberflächenbehandelten Glases (z.B. Glas GM 27884 k6, Fa. Schott, mit einem C-Wert von 2,1 %) zu einer Paste verarbeitet.

Vermischt man 10 Teile dieser Basispaste mit 1 Gewichtsteil Komponente B aus Beispiel 5, so härtet die Mischung in 10 Minuten zu einem elastischen Körper aus.

Gibt man diesen Körper 20 Minuten in kochendes Wasser, so erhält man einen Festkörper mit einer Biegefestigkeit von 69 N/mm², einem Biegemodul von 2700 N/mm².

## Patentansprüche (Patentansprüche für folgende(n) Vertragsstaat(en): AT, BE, CH, LI, DE, DK, FR, GB, GR, IT, LU, NL, SE)

1. Mehrstufig aushärtbare Kunststoffe, bestehend aus mindestens einem Silikopolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers.

2. Mehrstufig aushärtbare Kunststoffe, bestehend aus Ether-, Urethan- und Harnstoffgruppen enthaltenden Polyadditionsprodukten mit Alkoxysilylendgruppen einer überwiegend linearen Molekülstruktur mit ausschließlich aliphatisch oder cycloaliphatisch gebundenen Ether-, Urethan- und Harnstoffsegmenten und einem mittleren Molekulargewicht Mn von 800 - 20 000,
gekennzeichnet durch
a) einen Gehalt an Polyethergruppen von 25 bis 90 Gew.-Teilen, vorzugsweise von 50 bis 80 Gew.-Teilen auf 100 Gew.-Teile Polyadditionsprodukt,
b) einen Gehalt an Urethangruppen 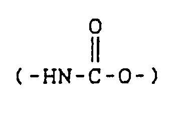 von 0,5 bis 10 Gew.-Teile, vorzugsweise von 1 bis 8 Gew.-Teilen auf 100 Gew.-Teile Polyadditionsprodukt,
c) einen Gehalt an Harnstoffgruppen 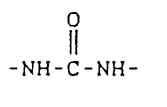 von 0,5 bis 10 Gew.-Teilen, vorzugsweise 1 - 8 Gew.-Teilen auf 100 Gew.-Teile Polyadditionsprodukt und
d) einen Gehalt an endständigen Alkoxysilylgruppen 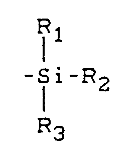 von 1 bis 25 Gew.-Teilen,
vorzugsweise 2 bis 10 Gew.-Teilen auf 100 Gew.-Teile Polyadditionsprodukt, wobei die Alkoxysilylgruppen im Polyaddukt über folgende Verbindungen eingebracht werden und folgende Formel aufweisen: 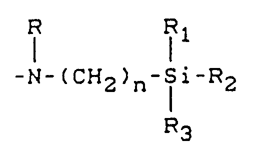 worin
n die Zahlen 1 bis 6, bevorzugt die Zahl 3 darstellt,
R Wasserstoff oder -(CH₂)ₙ-SiR₁R₂R₃ bedeutet,
R₁ C₁-C₄-Alkoxy, bevorzugt Methoxy oder Ethoxy bedeutet,
R₂ und R₃ die gleiche Bedeutung wie R₁ aufweisen und zusätzlich die Methyl- oder Ethylgruppe darstellen,
mindestens einem Methacrylat in monofunktioneller oder polyfunktioneller Form, mindestens einem Katalysator für die Heiß-, Kalt- oder Lichtpolymerisation und mindestens einem Katalysator zur Kondensation des Silikopolyethers.

3. Mehrstufig aushärtbare Kunststoffe nach den Ansprüchen 1 und 2, dadurch gekennzeichnet, daß die Methacrylate in der Mischung mit dem Silikopolyether zu 10 bis 90 Gew.-%, bezogen auf den Silikopolyether, enthalten sind.

4. Mehrstufig aushärtbare Kunststoffe nach den Ansprüchen 1 bis 3, dadurch gekennzeichnet, daß sie für die Heiß-, Kalt- und Lichtpolymerisation geeignete Katalysatoren in Mengen von 0,01 bis 10 Gew.-%, bezogen auf das polymerisierbare Material, enthalten.

5. Verwendung der mehrstufig härtbaren Kunststoffe nach den Ansprüchen 1 bis 4 im Dentalbereich zur Herstellung künstlicher Zähne oder Zahnteile.

6. Dentalartikel, hergestellt aus mehrstufig aushärtbaren Kunststoffen gemäß Anspruch 1.

7. Künstliche Zähne, hergestellt aus mehrstufig aushärtbaren Kunststoffen gemäß Anspruch 1.

8. Künstliche Zahnteile, hergestellt aus mehrstufig aushärtbaren Kunststoffen gemäß Anspruch 1.

## Patentansprüche (Patentansprüche für folgende(n) Vertragsstaat(en): ES)

1. Dentalartikel, hergestellt aus mindestens einem Silikopolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers.

2. Dentalartikel, hergestellt aus Ether-, Urethan- und Harnstoffgruppen enthaltenden Polyadditionsprodukten mit Alkoxysilylendgruppen einer überwiegend linearen Molekülstruktur mit ausschließlich aliphatisch oder cycloaliphatisch gebundenen Ether-, Urethan- und Harnstoffsegmenten und einem mittleren Molekulargewicht Mn von 800 - 20 000, gekennzeichnet durch
a) einen Gehalt an Polyethergruppen von 25 bis 90 Gew.-Teilen, vorzugsweise von 50 bis 80 Gew.-Teilen auf 100 Gew.-Teile Polyadditionsprodukt,
b) einen Gehalt an Urethangruppen 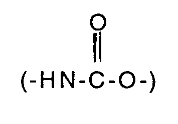 von 0,5 bis 10 Gew.-Teilen, vorzugsweise von 1 bis 8 Gew.-Teilen auf 100 Gew.-Teile Polyadditionsprodukt,
c) einen Gehalt an Harnstoffgruppen 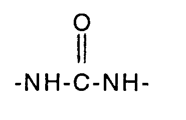 von 0,5 bis 10 Gew.-Teilen, vorzugsweise 1 - 8 Gew.-Teilen auf 100 Gew.-Teile Polyadditionsprodukt und
d) einen Gehalt an endständigen Alkoxysilylgruppen 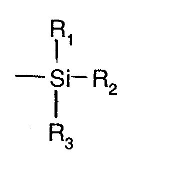 von 1 bis 25 Gew.-Teilen, vorzugsweise 2 bis 10 Gew.-Teilen auf 100 Gew.-Teile Polyadditionsprodukt, wobei die Alkoxysilylgruppen im Polyaddukt über folgende Verbindungen eingebracht werden und folgende Formel aufweisen: 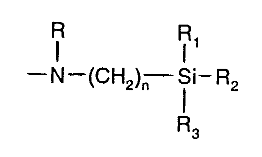 worin
n die Zahlen 1 bis 6, bevorzugt die Zahl 3 darstellt,
R Wasserstoff oder -(CH₂)ₙSiR₁R₂R₃ bedeutet,
R₁ C₁-C₄-Alkoxy, bevorzugt Methoxy oder Ethoxy bedeutet,
R₂ und R₃ die gleiche Bedeutung wie R₁ aufweisen und zusätzlich die Methyl- oder Ethylgruppe darstellen,
mindestens einem Methacrylat in monofunktioneller oder polyfunktioneller Form, mindestens einem Katalysator für die Heiß-, Kalt- oder Lichtpolymerisation und mindestens einem Katalysator zur Kondensation des Silikopolyethers.

3. Künstliche Zähne, hergestellt aus mindestens einem Silikopolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers.

4. Künstliche Zahnteile, hergestellt aus mindestens einem Silikopolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers.

5. Verwendung von Dentalartikeln, hergestellt aus mindestens einem Silikopolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers.

6. Verwendung von Dentalartikeln, hergestellt aus Ether-, Urethan- und Harnstoffgruppen enthaltenden Polyadditionsprodukten mit Alkoxysilylendgruppen einer überwiegend linearen Molekülstruktur mit ausschließlich aliphatisch oder cycloaliphatisch gebundenen Ether-, Urethan- und Harnstoffsegmenten und einem mittleren Molekulargewicht Mn von 800 - 20 000, gekennzeichnet durch
a) einen Gehalt an Polyethergruppen von 25 bis 90 Gew.-Teilen, vorzugsweise von 50 bis 80 Gew.-Teilen auf 100 Gew.-Teile Polyadditionsprodukt,
b) einen Gehalt an Urethangruppen 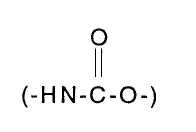 von 0,5 bis 10 Gew.-Teilen, vorzugsweise von 1 bis 8 Gew.-Teilen auf 100 Gew.-Teile Polyadditionsprodukt,
c) einen Gehalt an Harnstoffgruppen 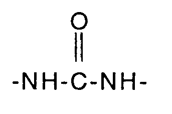 von 0,5 bis 10 Gew.-Teilen, vorzugsweise 1 - 8 Gew.-Teilen auf 100 Gew.-Teile Polyadditionsprodukt und
d) einen Gehalt an endständigen Alkoxysilylgruppen 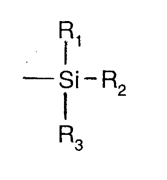 von 1 bis 25 Gew.-Teilen, vorzugsweise 2 bis 10 Gew.-Teilen auf 100 Gew.-Teile Polyadditionsprodukt, wobei die Alkoxysilylgruppen im Polyaddukt über folgende Verbindungen eingebracht werden und folgende Formel aufweisen: 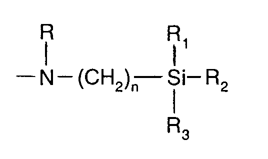 worin
n die Zahlen 1 bis 6, bevorzugt die Zahl 3 darstellt,
R Wasserstoff oder -(CH₂)ₙSiR₁R₂R₃ bedeutet,
R₁ C₁-C₄-Alkoxy, bevorzugt Methoxy oder Ethoxy bedeutet,
R₂ und R₃ die gleiche Bedeutung wie R₁ aufweisen und zusätzlich die Methyl- oder Ethylgruppe darstellen,
mindestens einem Methacrylat in monofunktioneller oder polyfunktioneller Form, mindestens einem Katalysator für die Heiß-, Kalt- oder Lichtpolymerisation und mindestens einem Katalysator zur Kondensation des Silikopolyethers.

## Claims (Claims for the following Contracting State(s): AT, BE, CH, LI, DE, DK, FR, GB, GR, IT, LU, NL, SE)

1. Plastics which are curable in a series of steps and which consist of at least one silicopolyether, at least one monomer which can be subjected to free-radical curing, and at least one catalyst for polymerization under the influence of high temperatures, low temperatures or light, and at least one catalyst for the condensation of the silicopolyether.

2. Plastics which are curable in a series of steps and which consist of polyaddition products, containing ether, urethane and urea groups and having alkoxysilyl terminal groups of a mostly linear molecular structure, with exclusively aliphatically or cycloaliphatically bonded ether, urethane and urea segments, and having a mean molecular weight Mn of 800 - 20,000,
characterized in that they contain
a) polyether groups in an amount of 25 to 90 parts by weight, preferably of 50 to 80 parts by weight, per 100 parts by weight of polyaddition product,
b) urethane groups 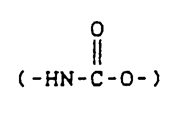 in an amount of 0.5 to 10 parts by weight, preferably of 1 to 8 parts by weight, per 100 parts by weight of polyaddition product,
c) urea groups 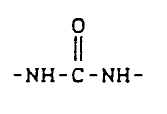 in an amount of 0.5 to 10 parts by weight, preferably 1 -8 parts by weight, per 100 parts by weight of polyaddition product, and
d) terminal alkoxysilyl groups 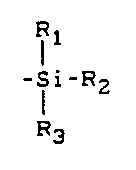 in an amount of 1 to 25 parts by weight, preferably 2 to 10 parts by weight, per 100 parts by weight of polyaddition product, the alkoxysilyl groups in the polyadduct being introduced by means of the following compounds and having the following formula: 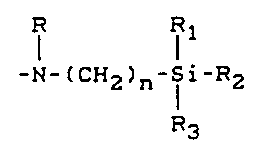 where
n represents the numbers 1 to 6, preferably the number 3,
R denotes hydrogen or -(CH₂)ₙ-SiR₁R₂R₃,
R₁ denotes C₁-C₄-alkoxy, preferably methoxy or ethoxy,
R₂ and R₃ have the same meaning as R₁ and additionally represent the methyl or ethyl group,
at least one methacrylate in monofunctional or polyfunctional form, at least one catalyst for polymerization under the influence of high temperatures, low temperatures or light, and at least one catalyst for the condensation of the silicopolyether.

3. Plastics which are curable in a series of steps, of Claims 1 and 2, characterized in that the methacrylates are contained in the mixture with the silicopolyether in amounts of 10 to 90 % by weight, based on the silicopolyether.

4. Plastics which are curable in a series of steps, of Claims 1 to 3, characterized in that they contain catalysts which are suitable for polymerization under the influence of high temperatures, low temperatures and light, in amounts of 0.01 to 10 % by weight, based on the polymerizable material.

5. Use of the plastics which are curable in a series of steps, of Claims 1 to 4, in the field of dental medicine, for making artificial teeth or parts of teeth.

6. Dental articles made from plastics which are curable in a series of steps, according to Claim 1.

7. Artificial teeth made from plastics which are curable in a series of steps, according to Claim 1.

8. Artificial parts of teeth made from plastics which are curable in a series of steps, according to Claim 1.

## Claims (Claims for the following Contracting State(s): ES)

1. Dental articles made from at least one silicopolyether, at least one monomer which can be subjected to free-radical curing, and at least one catalyst for polymerization under the influence of high temperatures, low temperatures or light, and at least one catalyst for the condensation of the silicopolyether.

2. Dental articles made from polyaddition products, containing ether, urethane and urea groups and having alkoxysilyl terminal groups of a mostly linear molecular structure, with exclusively aliphatically or cycloaliphatically bonded ether, urethane and urea segments, and having a mean molecular weight Mn of 800 - 20,000, characterized in that they contain
a) polyether groups in an amount of 25 to 90 parts by weight, preferably of 50 to 80 parts by weight, per 100 parts by weight of polyaddition product,
b) urethane groups 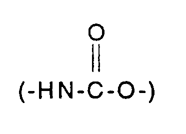 in an amount of 0.5 to 10 parts by weight, preferably of 1 to 8 parts by weight, per 100 parts by weight of polyaddition product,
c) urea groups 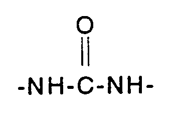 in an amount of 0.5 to 10 parts by weight, preferably 1 - 8 parts by weight, per 100 parts by weight of polyaddition product, and
d) terminal alkoxysilyl groups 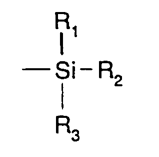 in an amount of 1 to 25 parts by weight, preferably 2 to 10 parts by weight, per 100 parts by weight of polyaddition product, the alkoxysilyl groups in the polyadduct being introduced by means of the following compounds and having the following formula: 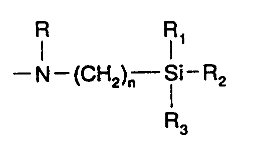 where
n represents the numbers 1 to 6, preferably the number 3,
R denotes hydrogen or -(CH₂)ₙSiR₁R₂R₃,
R₁ denotes C₁-C₄-alkoxy, preferably methoxy or ethoxy,
R₂ and R₃ have the same meaning as R₁ and additionally represent the methyl or ethyl group, at least one methacrylate in monofunctional or polyfunctional form, at least one catalyst for polymerization under the influence of high temperatures, low temperatures or light, and at least one catalyst for the condensation of the silicopolyether.

3. Artificial teeth made from at least one silicopolyether, at least one monomer which can be subjected to free-radical curing, and at least one catalyst for polymerization under the influence of high temperatures, low temperatures or light, and at least one catalyst for the condensation of the silicopolyether.

4. Artificial parts of teeth made from at least one silicopolyether, at least one monomer which can be subjected to free-radical curing, and at least one catalyst for polymerization under the influence of high temperatures, low temperatures or light, and at least one catalyst for the condensation of the silicopolyether.

5. Use of dental articles made from at least one silicopolyether, at least one monomer which can be subjected to free-radical curing, and at least one catalyst for polymerization under the influence of high temperatures, low temperatures or light, and at least one catalyst for the condensation of the silicopolyether.

6. Use of dental articles made from polyaddition products, containing ether, urethane and urea groups and having alkoxysilyl terminal groups of a mostly linear molecular structure, with exclusively aliphatically or cycloaliphatically bonded ether, urethane and urea segments, and having a mean molecular weight Mn of 800 - 20,000, characterized in that they contain
a) polyether groups in an amount of 25 to 90 parts by weight, preferably of 50 to 80 parts by weight, per 100 parts by weight of polyaddition product,
b) urethane groups 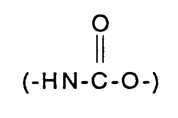 in an amount of 0.5 to 10 parts by weight, preferably of 1 to 8 parts by weight, per 100 parts by weight of polyaddition product,
c) urea groups 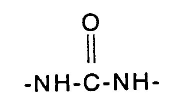 in an amount of 0.5 to 10 parts by weight, preferably 1 - 8 parts by weight, per 100 parts by weight of polyaddition product, and
d) terminal alkoxysilyl groups 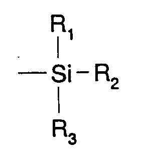 in an amount of 1 to 25 parts by weight, preferably 2 to 10 parts by weight, per 100 parts by weight of polyaddition product, the alkoxysilyl groups in the polyadduct being introduced by means of the following compounds and having the following formula: 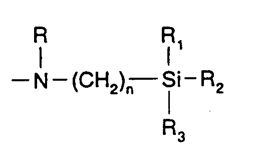 where
n represents the numbers 1 to 6, preferably the number 3,
R denotes hydrogen or -(CH₂)ₙSiR₁R₂R₃,
R₁ denotes C₁-C₄-alkoxy, preferably methoxy or ethoxy,
R₂ and R₃ have the same meaning as R₁ and additionally represent the methyl or ethyl group,
at least one methacrylate in monofunctional or polyfunctional form, at least one catalyst for polymerization under the influence of high temperatures, low temperatures or light, and at least one catalyst for the condensation of the silicopolyether.

## Revendications (Revendications pour l'(les) Etat(s) contractant(s) suivant(s): AT, BE, CH, LI, DE, DK, FR, GB, GR, IT, LU, NL, SE)

1. Matières plastiques durcissables en plusieurs étapes, constituées d'au moins un silicopolyéther, au moins un monomère durcissable par radicaux ainsi qu'au moins un catalyseur de polymérisation à chaud, à froid ou à la lumière et au moins un catalyseur de condensation du silicopolyéther.

2. Matières plastiques durcissables en plusieurs étapes, constituées de produits de polyaddition contenant des groupes éther, uréthanne et urée porteurs de groupes terminaux alkoxysilyle, de structure moléculaire principalement linéaire avec des segments éther, uréthanne et urée à liaison exclusivement aliphatique ou cycloaliphatique et ayant un poids moléculaire moyen Mn de 800 à 20 000,
caractérisées par
a) une teneur en groupes polyéther de 25 à 90 parties en poids, de préférence de 50 à 80 parties en poids pour 100 parties en poids de produit de polyaddition,
b) une teneur en groupes uréthanne 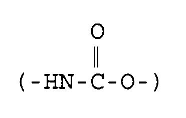 de 0,5 à 10 parties en poids, de préférence de 1 à 8 parties en poids pour 100 parties en poids de produit de polyaddition,
c) une teneur en groupes urée 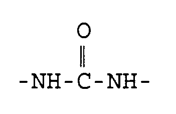 de 0,5 à 10 parties en poids, de préférence de 1 à 8 parties en poids pour 100 parties en poids de produit de polyaddition et
d) une teneur en groupes alkoxysilyle terminaux 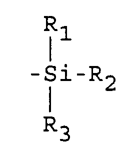 de 1 à 25 parties en poids, de préférence de 2 à 10 parties en poids pour 100 parties en poids de produit de polyaddition, les groupes alkoxysilyle présents dans le produit de polyaddition étant introduits par les composés suivants et présentant la formule suivante : 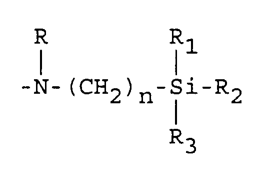 où
n représente des nombres de 1 à 6, de préférence le nombre 3,
R est de l'hydrogène ou un groupe -(CH₂)ₙSiR₁R₂R₃,
R₁ est un groupe alkoxy en C₁ à C₄, de préférence méthoxy ou éthoxy,
R₂ et R₃ présentent la même définition que R₁ et représentent en outre le groupe méthyle ou éthyle,
au moins un méthacrylate sous forme monofonctionnelle ou polyfonctionnelle, au moins un catalyseur de polymérisation à chaud, à froid ou à la lumière et au moins un catalyseur de condensation du silicopolyéther.

3. Matières plastiques durcissables en plusieurs étapes selon les revendications 1 et 2, caractérisées en ce que les méthacrylates sont contenus dans le mélange avec le silicopolyéther en proportion allant jusqu'à 10 à 90 % en poids par rapport au silicopolyéther.

4. Matières plastiques durcissables en plusieurs étapes suivant les revendications 1 à 3, caractérisées en ce qu'elles contiennent des catalyseurs qui conviennent pour la polymérisation à chaud, à froid et à la lumière, en quantités de 0,01 à 10 % en poids, par rapport à la matière polymérisable.

5. Utilisation de matières plastiques durcissables en plusieurs étapes suivant les revendications 1 à 4 dans le domaine dentaire pour la fabrication de dents ou de parties de dents artificielles.

6. Articles dentaires, fabriqués en matières plastiques durcissables en plusieurs étapes suivant la revendication 1.

7. Dents artificielles, fabriquées en matières plastiques durcissables en plusieurs étapes suivant la revendication 1.

8. Parties de dents artificielles, fabriquées en matières plastiques durcissables en plusieurs étapes suivant la revendication 1.

## Revendications (Revendications pour l'(les) Etat(s) contractant(s) suivant(s): ES)

1. Article dentaire, fabriqué à partir d'au moins un silicopolyéther, au moins un monomère durcissable par radicaux ainsi qu'au moins un catalyseur pour la polymérisation à chaud, à froid ou à la lumière et au moins un catalyseur de condensation du silicopolyéther.

2. Article dentaire, fabriqué à partir de produits de polyaddition contenant des groupes éther, uréthanne et urée porteurs de groupes terminaux alkoxysilyle, ayant une structure moléculaire principalement linéaire avec des segments éther, uréthanne et urée en liaison exclusivement aliphatique ou cycloaliphatique et ayant un poids moléculaire moyen Mn de 800 à 20 000, caractérisé par
a) une teneur en groupes polyéther de 25 à 90 parties en poids, de préférence de 50 à 80 parties en poids pour 100 parties en poids de produit de polyaddition,
b) une teneur en groupes uréthanne 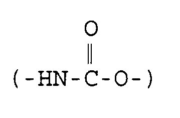 de 0,5 à 10 parties en poids, de préférence de 1 à 8 parties en poids pour 100 parties en poids de produit de polyaddition,
c) une teneur en groupes urée 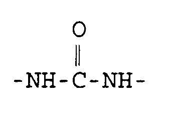 de 0,5 à 10 parties en poids, de préférence de 1 à 8 parties en poids pour 100 parties en poids de produit de polyaddition et
d) une teneur en groupes alkoxysilyle terminaux 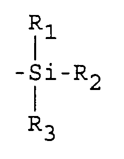 de 1 à 25 parties en poids, de préférence de 2 à 10 parties en poids pour 100 parties en poids de produit de polyaddition, les groupes alkoxysilyle présents dans le produit de polyaddition étant introduits par les composés suivants et présentant la formule suivante : 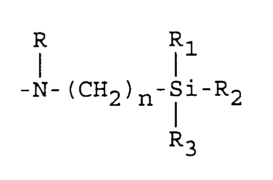 où
n représente des nombres de 1 à 6, de préférence le nombre 3,
R est de l'hydrogène ou un groupe -(CH₂)ₙSiR₁R₂R₃,
R₁ est un groupe alkoxy en C₁ à C₄, de préférence méthoxy ou éthoxy,
R₂ et R₃ présentent la même définition que R₁ et représentent en outre le groupe méthyle ou éthyle,
au moins un méthacrylate sous forme monofonctionnelle ou polyfonctionnelle, au moins un catalyseur de polymérisation à chaud, à froid ou à la lumière et au moins un catalyseur de condensation du silicopolyéther.

3. Dents artificielles, fabriquées à partir d'au moins un silicopolyéther, au moins un monomère durcissable par radicaux ainsi qu'au moins un catalyseur de polymérisation à chaud, à froid ou à la lumière et au moins un catalyseur de condensation du silicopolyéther.

4. Parties de dents artificielles, fabriquées à partir d'au moins un silicopolyéther, au moins un monomère durcissable par radicaux ainsi qu'au moins un catalyseur de polymérisation à chaud, à froid ou à la lumière et au moins un catalyseur de condensation du silicopolyéther.

5. Utilisation d'articles dentaires, fabriqués à partir d'au moins un silicopolyéther, au moins un monomère durcissable par radicaux ainsi qu'au moins un catalyseur de polymérisation à chaud, à froid ou à la lumière et au moins un catalyseur de condensation du silicopolyéther.

6. Utilisation d'articles dentaires, fabriqués en produits de polyaddition contenant des groupes éther, uréthanne et urée, porteurs de groupes terminaux alkoxysilyle, ayant une structure moléculaire principalement linéaire avec des segments éther, uréthanne et urée en liaison exclusivement aliphatique ou cycloaliphatique et ayant un poids moléculaire moyen Mn de 800 à 20 000, caractérisés par
a) une teneur en groupes polyéther de 25 à 90 parties en poids, de préférence de 50 à 80 parties en poids pour 100 parties en poids de produit de polyaddition,
b) une teneur en groupes uréthanne 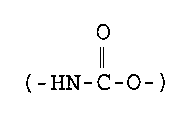 de 0,5 à 10 parties en poids, de préférence de 1 à 8 parties en poids pour 100 parties en poids de produit de polyaddition,
c) une teneur en groupes urée 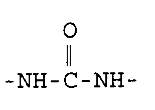 de 0,5 à 10 parties en poids, de préférence de 1 à 8 parties en poids pour 100 parties en poids de produit de polyaddition et
d) une teneur en groupes alkoxysilyle terminaux 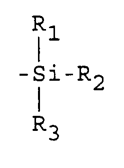 de 1 à 25 parties en poids, de préférence de 2 à 10 parties en poids pour 100 parties en poids de produit de polyaddition, les groupes alkoxysilyle présents dans le produit de polyaddition étant introduits par les composés suivants et présentant la formule suivante : 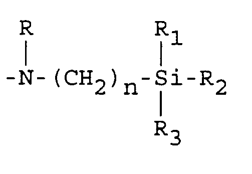 où
n représente des nombres de 1 à 6, de préférence le nombre 3,
R est de l'hydrogène ou un groupe -(CH₂)ₙSiR₁R₂R₃,
R₁ est un groupe alkoxy en C₁ à C₄, de préférence méthoxy ou éthoxy,
R₂ et R₃ présentent la même définition que R₁ et représentent en outre le groupe méthyle ou éthyle,
au moins un méthacrylate sous forme monofonctionnelle ou polyfonctionnelle, au moins un catalyseur de polymérisation à chaud, à froid ou à la lumière et au moins un catalyseur de condensation du silicopolyéther.
